# EUROPÄISCHE PATENTSCHRIFT

(11) **EP 0 918 753 B1**
(45) Veröffentlichungstag und Bekanntmachung des Hinweises auf die Patenterteilung: **14.01.2004**
(21) Anmeldenummer: 97925005.7
(22) Anmeldetag: 28.05.1997
(51) Int. Cl.: C07D 221/18, C09B 5/62

(54) **VERFAHREN ZUR HERSTELLUNG VON PERYLEN-3,4-DICARBONSÄUREIMIDEN**
PROCESS FOR PRODUCING PERYLENE-3,4-DICARBOXYLIC ACID IMIDES
PROCEDE DE PRODUCTION D'IMIDES D'ACIDE PERYLENE-3,4-DICARBOXYLIQUE

(30) Priorität: 05.06.1996 DE 19622673
(43) Veröffentlichungstag der Anmeldung: 02.06.1999
(73) Patentinhaber: BASF AKTIENGESELLSCHAFT, 67056 Ludwigshafen (DE)
(72) Erfinder: BÖHM, Arno, D-68305 Mannheim (DE); ARMS, Harald, D-67551 Worms (DE); HELFER, Willi, D-67159 Friedelsheim (DE); HENNING, Georg, D-67061 Ludwigshafen (DE)
(86) Internationale Anmeldenummer: PCT/EP1997/002761
(87) Internationale Veröffentlichungsnummer: WO 1997/046533

(56) Entgegenhaltungen:
- EP-A- 0 657 436
- WO-A-96/22331
- DE-A- 4 338 784
- DE-A- 4 440 242
- FEILER ET AL.: "Synthesis of Perylene-3,4-dicarboximides ..." LIEBIGS ANN., 1995, Seiten 1229-1244, XP002040181
- 'HUBEN-WEIL', Bd. E5, 1985, GEORG THIME VERLAG, STUTTGART Seiten 1118 - 1122

## Beschreibung

Die vorliegende Erfindung betrifft ein neues Verfahren zur Herstellung von Perylen-3,4-dicarbonsäureimiden der allgemeinen Formel I 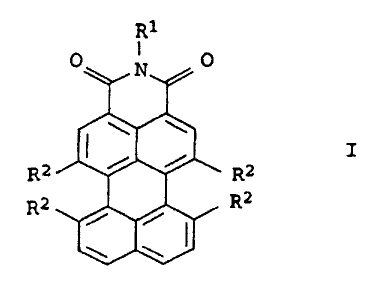 in der die Variablen folgende Bedeutung haben:
- R¹: Wasserstoff; C₁-C₈-Alkyl; C₅-C₈-Cycloalkyl; Phenyl oder Naphthyl, das jeweils durch C₁-C₁₀-Alkyl, C₁-C₆-Alkoxy oder Cyano substituiert sein kann;
- R²: unabhängig voneinander Wasserstoff; C₁-C₁₈-Alkyl; Aryloxy, Arylthio, Hetaryloxy oder Hetarylthio, das jeweils durch C₁-C₁₀-Alkyl, C₁-C₆-Alkoxy oder Cyano substituiert sein kann.

Perylen-3,4-dicarbonsäureimide der Formel I sind bekanntermaßen wichtige Zwischenprodukte für die Herstellung von Pigmentadditiven, Fluoreszenzfarbstoffen und Fluoreszenzpigmenten (EP-A-636 666; EP-A-596 292), sie eignen sich jedoch vorteilhaft auch selbst als Fluoreszenzfarbstoffe und -pigmente (EP-A-657 436; nicht vorveröffentlichte DE-A-195 01 737).

Zur Herstellung von im Perylengerüst unsubstituierten Perylen-3,4-dicarbonsäureimiden I (R²=H) sind eine Reihe von aufwendigen, mehrstufigen Verfahren beschrieben, die von Perylen-3,4,9,10-tetracarbonsäuredianhydrid ausgehen und die Verbindungen I in zumeist wenig befriedigenden Ausbeuten und so schlechten Reinheiten liefern, daß aufwendige Reinigungsmethoden wie Extraktion und Säulenchromatographie erforderlich werden.

So werden unsubstituiertes und N-alkylsubstituierte Perylen-3,4-dicarbonsäureimide (Alkyl: Methyl, Ethyl, n-Propyl, n-Butyl, Isobutyl, n-Pentyl, n-Hexyl, n-Octyl, n-Dodecyl) durch alkalische Decarboxylierung der als Zwischenstufe hergestellten Perylen-3,4,9,10-tetracarbonsäureimidanhydride bei Temperaturen ≥ 220°C unter Druck (Reaktionszeiten von 18 h) erhalten (DE-PS-486 491; Bulletin of the Chemical Society of Japan 54, 1575-1576 (1981); EP-A-596 292). Dieses verfahren ist jedoch nur für gegenüber Basen stabile aliphatische Imide geeignet.

Zur Herstellung der N-methyl- und -ethylsubstituierten (sowie auch N-phenyl-, -tolyl- und -anisylsubstituierten) Perylen-3,4-dicarbonsäureimide wird das zunächst hergestellte unsubstituierte Perylen-3,4-dicarbonsäureimid mit Oleum sulfoniert, anschließend mit Kalilauge in das sulfonierte Anhydrid überführt, welches dann mit dem entsprechenden primären Amin zu dem sulfonierten, N-substituierten Imid umgesetzt wird, welches schließlich mit Schwefelsäure zum gewünschten Perylen-3,4-dicarbonsäureimid desulfoniert wird (Bulletin of the Chemical Society of Japan 52, 1723-1726 (1979), Shikizai Kyokaishi 49, 29-34 (1976) 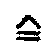 Chemical Abstracts 85:209285). Dieses Verfahren ist sehr umständlich und kann nur für gegenüber Schwefelsäure stabile Imide eingesetzt werden.

Nach der EP-A-657 436 werden unsubstituierte, N-alkyl-, N-cycloalkyl- und N-phenylsubstituierte Perylen-3,4-dicarbonsäureimide mit unsubstituiertem Perylengerüst durch Kondensation von Perylen-3,9,9,10-tetracarbonsäuredianhydrid mit 2,5-Di-tert.-butylanilin in Gegenwart von Wasser, Zinkacetatdihydrat und Imidazol, alkalische Verseifung des chromatographisch gereinigten N-2,5-Di-tert.-butylphenylperylendicarbonsäureimids zum Perylen-3,4-dicarbonsäureanhydrid und dessen Umsetzung mit dem entsprechenden primären Amin erhalten.

Ein weiteres in der EP-A-657 436 beschriebenes Verfahren zur Herstellung N-alkylsubstituierter Perylen-3,4-dicarbonsäureimide geht vom unsubstituierten Perylen-3,4-dicarbonsäureimid aus, das zunächst, wie oben beschrieben, aus Perylen-3,4,9,10-tetracarbonsäuredianhydrid herzustellen ist und mit dem entsprechenden Alkylbromid in Gegenwart einer starken Base und eines dipolar-aprotischen Lösungsmittels umgesetzt wird. Die Reaktionsprodukte werden hier ebenfalls einer Chromatographie unterzogen.

Schließlich können N-1-Hexylheptyl- und N-1-Octylnonylperylen-3,4-dicarbonsäureimid nach der EP-A-657 436 analog zu dem als Zwischenprodukt hergestellten N-2,5-Di-tert.-butylphenylperylen-3,4-dicarbonsäureimid auch direkt aus Perylen-3,4,9,10-tetracarbonsäuredianhydrid erhalten werden.

Aber auch die aus der EP-A-657 436 bekannten Herstellungsverfahren sind sehr aufwendig und ergeben die N-substituierten Perylen-3,4-dicarbonsäureimide nur in geringen Gesamtausbeuten.

Ein Verfahren, das auch zur Herstellung von im Perylengerüst substituierten Perylen-3,4-dicarbonsäureimiden geeignet ist, wird in der nicht vorveröffentlichten DE-A-195 01 737 beschrieben. Die Imide I wurden hier durch direkte Umsetzung von Perylen-3,4,9,10-tetracarbonsäuredianhydrid und primärem Amin in Gegenwart einer tertiären stickstoffbasischen Verbindung wie Chinolin und von Zink, Kupfer oder deren Salzen als Katalysator ohne Zusatz von Wasser erhalten.

Der Erfindung lag die Aufgabe zugrunde, ein einfaches und wirtschaftliches Verfahren bereitzustellen, welches die oben genannten Nachteile nicht aufweist und die Herstellung von Perylen-3,4-dicarbonsäureimiden, die am Imidstickstoff und/oder im Perylengerüst substituiert sein können, in guten Ausbeuten und für den weiteren Verwendungszweck ausreichenden Reinheiten ermöglicht.

Demgemäß wurde ein Verfahren zur Herstellung von Perylen-3,4-dicarbonsäureimiden der allgemeinen Formel I 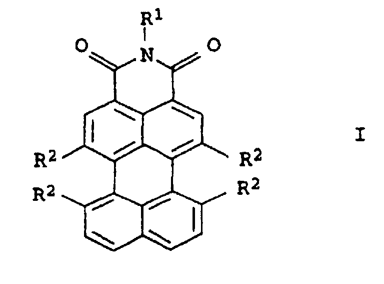 in der die Variablen folgende Bedeutung haben:
- R¹: Wasserstoff; C₁-C₈-Alkyl; C₅-C₈-Cycloalkyl; Phenyl oder Naphthyl, das jeweils durch C₁-C₁₀-Alkyl, C₁-C₆-Alkoxy oder Cyano substituiert sein kann;
- R²: unabhängig voneinander Wasserstoff; C₁-C₁₈-Alkyl; Aryloxy, Arylthio, Hetaryloxy oder Hetarylthio, das jeweils durch C₁-C₁₀-Alkyl, C₁-C₆-Alkoxy oder Cyano substituiert sein kann,
gefunden, welches dadurch gekennzeichnet ist, daß man eine Perylen-3,4,9,10-tetracarbonsäure bzw. deren Anhydrid der Formel II 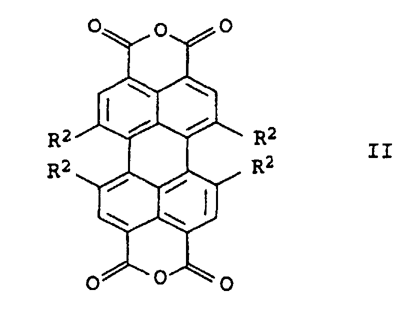 mit einem Amid der Formel III

R³ ― CO― NHR¹ III

in der R³ für C₁-C₄-Alkyl oder für einen Rest -NR⁴R⁵ steht, wobei, R⁴ und R⁵ unabhängig voneinander Wasserstoff oder einen der für R² definierten Alkyl-, Cycloalkyl-, Phenyl- oder Naphthylreste bedeuten und wobei, für den Fall, daß mehr als einer der Reste R¹, R⁴ und/oder R⁵ ungleich Wasserstoff ist, diese Reste die gleiche Bedeutung haben,
in Gegenwart eines inerten Verdünnungsmittels und eines Übergangsmetallkatalysators unter Druck umsetzt.

Die in den Formeln I, II und III auftretenden Alkylgruppen können sowohl geradkettig als auch verzweigt sein. Aromatische Reste, die substituiert sind, können im allgemeinen bis zu 3, bevorzugt 1 oder 2 der genannten Substituenten aufweisen.

Als Beispiele für geeignete Reste R¹ sowie R² oder R³, R⁴ oder R⁵ (bzw. für deren Substituenten) seien im einzelnen genannt:
Methyl, Ethyl, Propyl, Isopropyl, Butyl, Isobutyl, tert.-Butyl, Pentyl, Isopentyl, Neopentyl, tert.-Pentyl, Hexyl, 2-Methylpentyl, Heptyl, 1-Ethylpentyl, Octyl. 2-Ethylhexyl, Isooctyl, Nonyl, Isononyl, Decyl, Isodecyl, Undecyl, Dodecyl, Tridecyl, Isotridecyl, Tetradecyl, Pentadecyl, Hexadecyl, Heptadecyl und Octadecyl (die obigen Bezeichnungen Isooctyl, Isononyl, Isodecyl und Isotridecyl sind Trivialbezeichnungen und stammen von den nach der Oxosynthese erhaltenen Alkoholen - vgl. dazu Ullmanns Encyklopädie der technischen Chemie, 4. Auflage, Band 7, Seiten 215 bis 217, sowie Band 11, Seiten 435 und 436);
Methoxy, Ethoxy, Propoxy, Isopropoxy, Butoxy, Isobutoxy, tert.-Butoxy, Pentoxy, Isopentoxy, Neopentoxy, tert.-Pentoxy und Hexoxy;
Phenyl, 2-Naphthyl, 2-, 3- und 4-Methylphenyl, 2,4-, 3,5- und 2,6-Dimethylphenyl, 2,4,6-Trimethylphenyl, 2-, 3- und 4-Ethylphenyl, 2,4-, 3,5- und 2,6-Diethylphenyl, 2,4,6-Triethylphenyl, 2-, 3- und 4-Propylphenyl, 2,4-, 3,5- und 2,6-Dipropylphenyl, 2,4,6-Tripropylphenyl, 2-, 3- und 4-Isopropylphenyl, 2,4-, 3,5- und 2,6-Diisopropylphenyl, 2,4,6-Triisopropylphenyl, 2-, 3- und 4-Butylphenyl, 2,4-, 3,5- und 2,6-Dibutylphenyl, 2,4,6-Tributylphenyl, 2-, 3- und 4-Isobutylphenyl, 2,4-, 3,5- und 2,6-Diisobutylphenyl, 2,4,6-Triisobutylphenyl, 2-, 3- und 4-sec.-Butylphenyl, 2,4-, 3,5- und 2,6-Di-sec.-butylphenyl und 2,4,6-Tri-sec.-butylphenyl; 2-, 3- und 4-Methoxyphenyl, 2,4-, 3,5- und 2,6-Dimethoxyphenyl, 2,4,6-Trimethoxyphenyl, 2-, 3- und 4-Ethoxyphenyl, 2,4-, 3,5- und 2,6-Diethoxyphenyl, 2,4,6-Triethoxyphenyl, 2-, 3- und 4-Propoxyphenyl, 2,4-, 3,5- und 2,6-Dipropoxyphenyl, 2-, 3- und 4-Isopropoxyphenyl, 2,4- und 2,6-Diisopropoxyphenyl und 2-, 3- und 4-Butoxyphenyl; 2-, 3- und 4-Cyanophenyl;
Phenoxy, Phenylthio, 2-Naphthoxy, 2-Naphthylthio, 2-, 3- und 4-Pyridyloxy, 2-, 3- und 4-Pyridylthio, 2-, 4- und 5-Pyrimidyloxy und 2-, 4- und 5-Pyrimidylthio.

Beim erfindungsgemäBen Verfahren zur Herstellung der Perylen-3,4-dicarbonsäureimide I werden die Perylen-3,4,9,10-tetracarbonsäuredianhydride II (bzw. die entsprechenden Säuren) nicht wie bei den üblichen Imidierungsreaktionen mit primären Aminen, sondern mit Amiden der Formel III als Aminäquivalenten umgesetzt, die eine gezielte Steuerung der Umsetzung, insbesondere auch eine weitgehende Unterdrückung der störenden Nebenreaktion zu den ungewünschten Perylentetracarbonsäurediimiden, ermöglichen und auch die Herstellung der N-C₁-C₄-Alkylimide I und der unsubstituierten Imide I vorteilhaft erlauben.

Als Amide III eignen sich dabei sowohl die entsprechenden N-substituierten oder unsubstituierten Amide (R¹=H) von aliphatischen C₁-C₄-Carbonsäuren wie insbesondere Ameisen- und Essigsäure (Fall a) als auch Harnstoff (R¹=H) und die entsprechenden N-monosubstituierten, N,N- und N,N'-disubstituierten und N,N,N'-trisubstituierten Harnstoffderivate (R¹≠ H; Fall b). Bevorzugt sind solche Amide III, die in dem verwendeten Verdünnungsmittel zumindest teilweise löslich sind, da sie dem Reaktionsgemisch vorteilhaft während der Umsetzung als Lösung gezielt (kontinuierlich oder auch diskontinuierlich) zugesetzt werden können.

Die Carbonsäureamide IIIa werden vorzugsweise zur Herstellung von Perylen-3,4-dicarbonsäureimiden der Formel I, in der R¹ (insbesondere unverzweigtes) C₁-C₄-Alkyl oder vor allem Wasserstoff oder Aryl bedeutet, eingesetzt.

Die Harnstoffderivate IIIb, insbesondere die di- und vor allem die monosubstituierten Derivate, können vorteilhaft zur Herstellung von sämtlichen Perylen-3,4-dicarbonsäureimiden I, auch von solchen mit verzweigten und/oder höheren Alkyl-, Cycloalkyl- und Arylresten R¹, verwendet werden.

Als Beispiele für besonders geeignete Amide III seien im einzelnen genannt: Formamid, Acetamid, N-Methyl-, N-Ethyl-, N-n-Propylund N-n-Butyl-, N-Phenyl-, N-o-Tolyl- und N-p-Tolylformamid und -acetamid, Harnstoff, N-Methyl-, N-Ethyl-, N-Propyl-, N-Butyl-, N-Pentyl-, N-Hexyl-, N-Heptyl- und N-Octylharnstoff, N,N- und N,N'-Dimethyl- und N,N- und N,N'-Diethylharnstoff, N-Cyclopentyl-, N-Cyclohexyl-, N-Cycloheptyl-, N-Cyclooctyl-, N-Phenyl-, N-o-Tolyl-, N-p-Tolyl- und N-2-Naphthylharnstoff.

Beim Einsatz der Carbonsäureamide IIIa beträgt das Molverhältnis von Perylen-3,4,9,10-tetracarbonsäuredianhydrid II zu IIIa in der Regel 0,5 : 1 bis 1,2 : 1, vorzugsweise 0,9 : 1 bis 1,1 : 1.

Bei Verwendung von Harnstoff und seinen Derivaten IIIb liegt das Molverhältnis von II zu IIIb im allgemeinen bei 0,9 : 1 bis 2,5 : 1, bei mono- und trisubstituierten Harnstoffen bevorzugt bei 0,9 : 1 bis 1,1 : 1 und bei Harnstoff selbst und disubstituierten Harnstoffen bevorzugt bei 1,8 : 1 bis 2,2 : 1.

Die erfindungsgemäße Umsetzung der Perylen-3,4,9,10-tetracarbonsäuredianhydride II (bzw. der entsprechenden Säuren) mit den Amiden III wird in Gegenwart eines inerten Verdünnungsmittels und eines Übergangsmetallkatalysators unter Druck vorgenommen.

Als Verdünnungsmittel eignen sich dabei bevorzugt tertiäre Stickstoffbasen, z.B. cyclische Imide wie N-Methylpyrrolidon, tertiäre aliphatische Amine NR₃, deren Alkylreste R 4 bis 8 C-Atome aufweisen, wie Trihexylamin, und vor allem aromatische Heterocyclen wie Pyridin, Chinaldin, Isochinolin und insbesondere Chinolin.

Die Menge an Verdünnungsmittel ist an sich nicht kritisch, sie beträgt üblicherweise 2 bis 12 kg, vorzugsweise 4 bis 8 kg, je kg Perylen-3,4,9,10-tetracarbonsäuredianhydrid II.

Als Übergangsmetallkatalysatoren eignen sich neben Zink und seinen Salzen vor allem Kupfer und seine anorganischen und organischen Salze, die vorzugsweise in wasserfreier Form eingesetzt werden.

Beispiele für bevorzugte Salze sind Kupfer(I)oxid, Kupfer(II)oxid, Kupfer(I)chlorid, Kupfer (II) chlorid, Kupfer(I)bromid, Kupfer(II)bromid, Kupfer (II) acetat, Kupfer (II) acetylacetonat, basisches Kupfer(II)carbonat und Kupfer (II) sulfat.

Selbstverständlich kann man auch Mischungen der genannten Katalysatoren verwenden.

In der Regel kommen 20 bis 150 Gew.-%, vorzugsweise 25 bis 60 Gew.-%, Katalysator, bezogen auf das Perylen-3,4,9,10-tetracarbonsäuredianhydrid II, zum Einsatz.

Das erfindungsgemäße Herstellungsverfahren wird zweckmäßigerweise in einem geschlossenen Rührdruckkessel (Autoklav) unter Eigendruck durchgeführt. Im Verlauf der Umsetzung kann sich dabei abhängig von den gewählten Reaktionsbedingungen (z.B. der Reaktionstemperatur von in der Regel 120 bis 250°C, vorzugsweise 160 bis 200°C) ein Druck von bis zu 25 bar einstellen. Bevorzugt sind jedoch Drücke von bis zu 18 bar.

Es empfiehlt sich, unter Verwendung einer Schutzgasatmosphäre (z.B. Stickstoff) zu arbeiten.

Üblicherweise ist die erfindungsgemäße Umsetzung in 2 bis 30 h, insbesondere 3 bis 12 h, beendet.

Verfahrenstechnisch kann man beim erfindungsgemäßen Verfahren so vorgehen, daß man das Amid III dem Reaktionsgemisch vor dem Erhitzen auf Reaktionstemperatur auf einmal zugibt oder das in einem Teil des eingesetzten Verdünnungsmittels gelöste Amid III dem auf Reaktionstemperatur erhitzten Reaktionsgemisch (vorzugsweise kontinuierlich) zudosiert.

Bei der erstgenannten Arbeitsweise geht man zweckmäßigerweise wie folgt vor:
Man legt Perylen-3,4,9,10-tetracarbonsäuredianhydrid II, Katalysator und Amid III in der gesamten Verdünnungsmittelmenge vor und spült die Druckapparatur mit Stickstoff (etwa 15 min). Nach Verschließen der Apparatur erhitzt man das Gemisch unter Rühren auf die Reaktionstemperatur und rührt es etwa 3 bis 8 h bei dieser Temperatur.

Nach Abkühlen auf üblicherweise 20 bis 50°C und Entspannen kann man das Reaktionsgemisch nach einer der folgenden Varianten aufarbeiten:
Bei der einen Aufarbeitungsvariante geht man im allgemeinen so vor, daß man das Reaktionsgemisch mit etwa der 1- bis 1,5-fachen Menge Alkohol, vorzugsweise Methanol, versetzt, und das ausgefallene Rohprodukt abfiltriert. Nicht umgesetztes Perylen-3,4,9,10-tetracarbonsäuredianhydrid II kann man anschließend durch 0,5 bis lstündiges Auf rühren des Rohprodukts in etwa der 6- bis 10-fachen Menge an heißer, verdünnter, anorganischer Base (z.B. 10 gew.-%iger Kaliumcarbonatlösung oder 5 bis 10 gew.-%iger Kalilauge), erneutes Abfiltrieren und Waschen zunächst bis zum farblosen Ablauf mit heißer wäßriger Base und dann bis zur Neutralität des ablaufenden waschwassers mit Wasser entfernen.
Bei der anderen Aufarbeitungsvariante geht man in der Regel so vor, daß man das Reaktionsgemisch mit etwa der 1- bis 1,5-fachen Menge Alkylacetat, vor allem C₁-C₄-Alkylacetat, insbesondere Ethylacetat, versetzt, das ausgefallene Produkt abfiltriert und es zunächst mit weiterem Alkylacetat bis zum farblosen Ablauf und dann mit Wasser lösungsmittelfrei wäscht.

Zur vollständigen Entfernung des Katalysators kann man den Filterkuchen bei beiden Aufarbeitungsvarianten anschließend etwa 0,5 bis 1 h in verdünnter anorganischer Säure (z.B. 10 bis 15 gew.-%iger Salzsäure) kochen.

Danach kann man die Imide I wie üblich durch Filtration der abgekühlten Mischung, Waschen mit Wasser bis zur Neutralität und Salzfreiheit des ablaufenden Waschwassers und Trocknen isolieren.

Bei der zweitgenannten Arbeitsweise geht man zweckmäßigerweise so vor, daß man Perylen-3,4,9,10-tetracarbonsäuredianhydrid II und Katalysator nur in einem Teil der Verdünnungsmittelmenge (z.B. etwa der Hälfte) vorlegt, dann, wie oben beschrieben, das Gemisch nach Spülen mit Stickstoff und Verschließen der Apparatur auf Reaktionstemperatur erhitzt und anschließend das in der restlichen Verdünnungsmittelmenge gelöste Amid III bei konstantem Volumenfluß in etwa 4 bis 8 h zudosiert. Nach einer Nachrührzeit von etwa 0,5 bis 8 h kühlt man das Reaktionsgemisch auf üblicherweise 20 bis 50°C ab und entspannt. Die weitere Aufarbeitung kann man ebenfalls wie oben beschrieben vornehmen.

In der Regel haben die so behandelten Produkte I bereits einen so hohen Wertgehalt (>80 %), daß eine weitere Reinigung nicht erforderlich ist.

Will man den Wertgehalt jedoch bis auf >98 % steigern, so kann man die Perylen-3,4-dicarbonsäureimide I noch dem in der nicht vorveröffentlichten DE-A-195 01 737 beschriebenen Reinigungsverfahren unterziehen, indem man sie zunächst in N-Methylpyrrolidon erhitzt und die gebildeten N-Methylpyrrolidon-Addukte dann in Gegenwart eines organischen Verdünnungsmittels (insbesondere eines aliphatischen Alkohols wie Isopropanol) in der Wärme mit einer Base (insbesondere einem wäßrigen Alkalimetallhydroxid) behandelt und die danach isolierten Produkte gewünschtenfalls noch einer zusätzlichen Behandlung mit einer verdünnten anorganischen Säure (insbesondere Salzsäure) unterzieht.

Mit Hilfe des erfindungsgemäßen Herstellungsverfahrens können sowohl im Perylengerüst substituierte als auch unsubstituierte Perylen-3,4-dicarbonsäureimide vorteilhaft in hohen Ausbeuten (in der Regel >70 %) und guten Reinheiten auf einfache, wirtschaftliche Weise hergestellt werden. Insbesondere können auch die am Imid-Stickstoff unsubstituierten oder durch niedriges Alkyl (C₁-C₄-Alkyl, besonders Methyl) substituierten Perylen-3,4-dicarbonsäureimide I in einfacher Weise erhalten werden.

### Beispiele

Herstellung von Perylen-3,4-dicarbonsäureimiden der Formel Ia 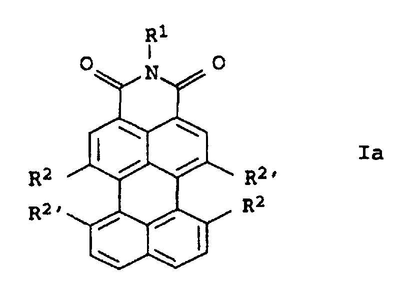 aus Perylen-3,4,9,10-tetracarbonsäuredianhydriden der Formel IIa

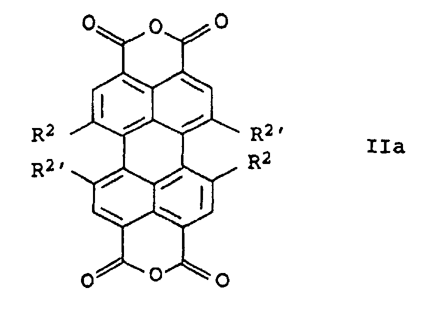

### Beispiele 1 bis 17

### Verfahrensvariante 1 (V1)

In einem 1 l-Rührdruckkessel wurde eine Mischung von 0,15 mol des Perylen-3,4,9,10-tetracarbonsäuredianhydrids IIa, 0,15 mol des Amids III (Beispiel 1: 0,075 mol Harnstoff; Beispiele 5 bis 7: 0,075 mol Dimethylharnstoff) und x g des Katalysators K in 300 ml Chinolin 15 min mit Stickstoff gespült. Nach druckdichtem Verschließen des Kessels wurde der Kesselinhalt unter Rühren auf T°C erhitzt und t h bei dieser Temperatur gehalten.

Nach Abkühlen auf Raumtemperatur und Entspannen wurde das Reaktionsgemisch mit 400 ml Methanol versetzt. Das auf diese Weise ausgefällte Rohprodukt wurde über eine G3-Glasfritte abfiltriert und mit Methanol chinolinfrei gewaschen.

Zur weiteren Reinigung wurde das Rohprodukt in 500 ml heißer 10 gew.-%iger wäßriger Kaliumcarbonatlösung aufgeschlämmt, 1 h bei 80°C gerührt, erneut abfiltriert und zunächst mit heißer Kaliumcarbonatlösung bis zum farblosen Ablauf und dann mit Wasser neutral gewaschen. Zur vollständigen Entfernung des Katalysators wurde der Filterkuchen anschließend 1 h in 500 ml 10 gew.-%iger Salzsäure gekocht, abfiltriert, mit Wasser neutral und salzfrei gewaschen und getrocknet.

### Verfahrensvariante 2 (V2)

In einem 1 l-Rührdruckkessel wurde eine Mischung von 0,15 mol des Perylen-3,4,9,10-tetracarbonsäuredianhydrids IIa und x g des Katalysators K in 250 ml Chinolin 15 min mit Stickstoff gespült. Nach druckdichtem Verschließen des Kessels wurde der Kesselinhalt unter Rühren auf T°C erhitzt. Bei dieser Temperatur wurde eine Lösung von 0,15 mol des Amids III in 50 ml Chinolin (Beispiel 8: + 20 ml N-Methylpyrrolidon zur Steigerung der Löslichkeit) in t₁ h kontinuierlich über eine Pumpe zudosiert.

Nach einer Nachrührzeit von t₂ h bei T°C wurde das Reaktionsgemisch auf Raumtemperatur abgekühlt, entspannt und wie bei der Verfahrensvariante V1 beschrieben aufgearbeitet.

Weitere Einzelheiten zu diesen Versuchen sowie deren Ergebnisse einschließlich der über quantitative Dünnschichtchromatographie auf Kieselgel gegen hochreine Standards mit einer Trichloressigsäure/Toluol-Mischung (1:3 bis 1:10 v/v) als Eluens ermittelten Wertgehalte sind in der folgenden Tabelle zusammengestellt.

### Anmerkung zu Beispiel 11:

Das eingesetzte 1,7-Diphenoxyperylen-3,4,9,10-tetracarbonsäuredianhydrid wurde, wie in der älteren deutschen Patentanmeldung 195 47 209.8 beschrieben, durch Bromierung von Perylen-3,4,9,10-tetracarbonsäuredianhydrid, Umsetzung des gebildeten 1,7-Dibromperylen-3,4,9,10-tetracarbonsäuredianhydrids mit Cyclohexylamin, Umsetzung des bei der Imidierung gebildeten N,N'-Dicyclohexyl-1,7-dibromperylen-3,4,9,10-tetracarbonsäurediimids mit Phenol und anschließende Verseifung des N,N'-Dicyclohexyl-1,7-diphenoxyperylen-3,4,9,10-tetracarbonsäurediimids erhalten.

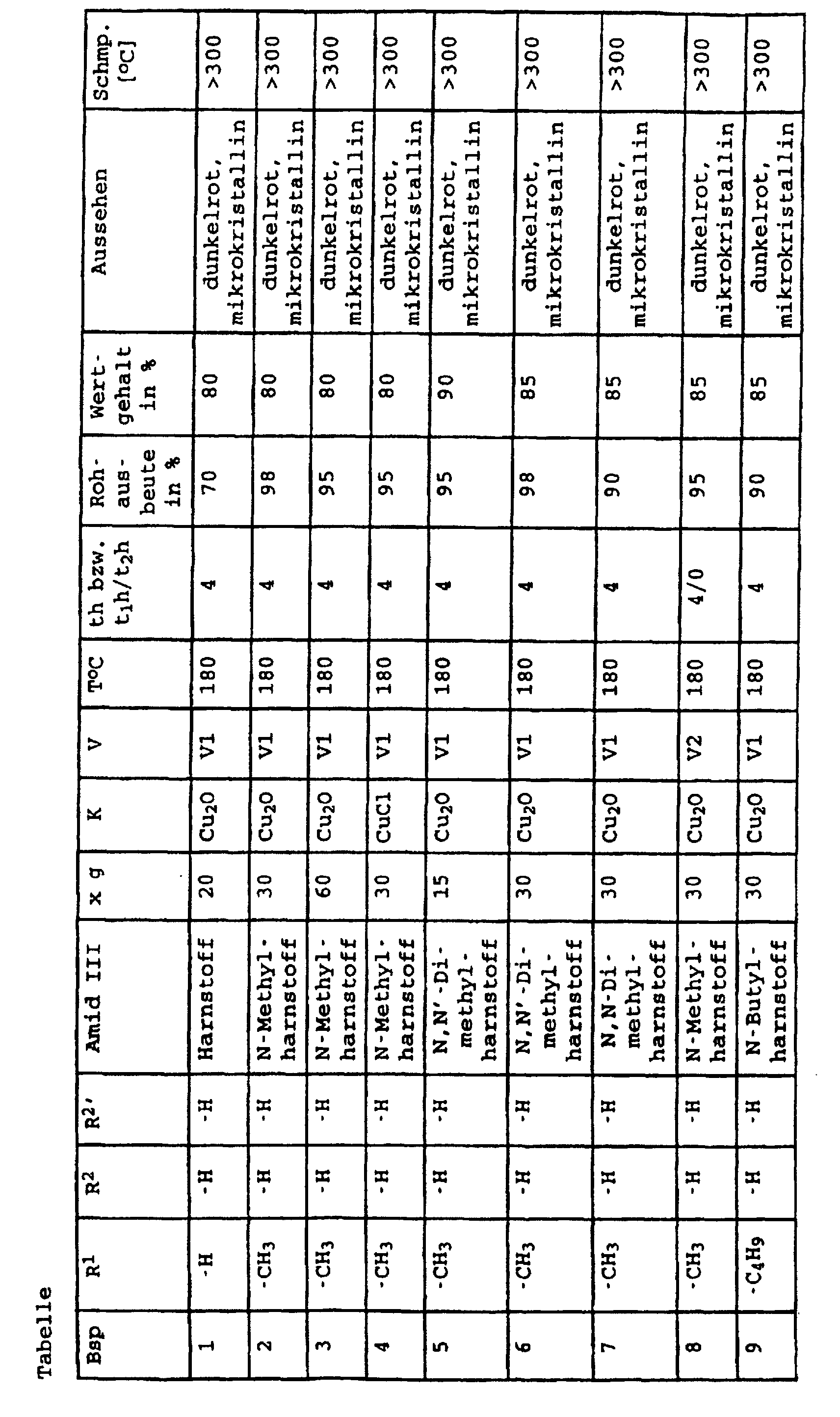

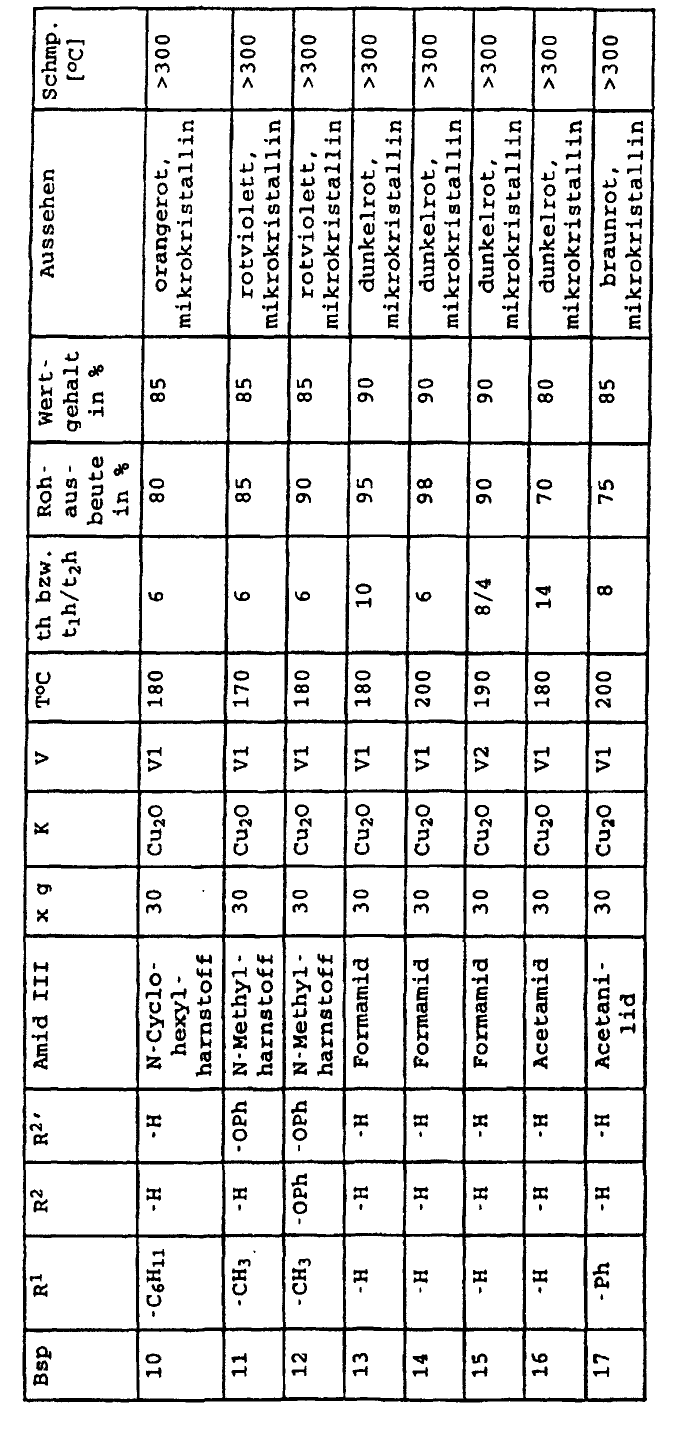

## Patentansprüche

1. verfahren zur Herstellung von Perylen-3,4-dicarbonsäureimiden der allgemeinen Formel 1 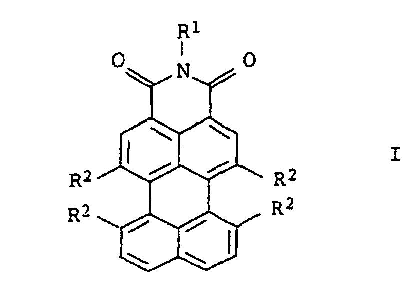 in der die Variablen folgende Bedeutung haben:
R¹ Wasserstoff; C₁-C₈-Alkyl; C₅-C₈-Cycloalkyl; Phenyl oder Naphthyl, das jeweils durch C₁-C₁₀-Alkyl, C₁-C₆-Alkoxy oder Cyano substituiert sein kann;
R² unabhängig voneinander Wasserstoff; C₁-C₁₈-Alkyl; Aryloxy, Arylthio, Hetaryloxy oder Hetarylthio, das jeweils durch C₁-C₁₀-Alkyl, C₁-C₆-Alkoxy oder Cyano substituiert sein kann,
**dadurch gekennzeichnet, daß** man eine Perylen-3,4,9,10-tetracarbonsäure bzw. deren Anhydrid der Formel II 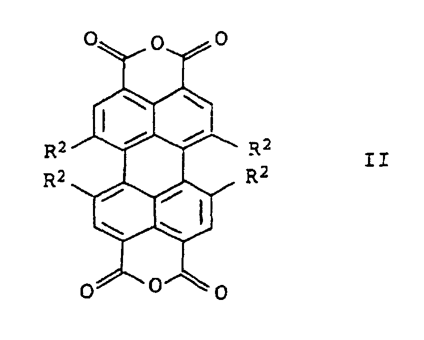 mit einem Amid der Formel III
R³― CO ― NHR¹ III
in der R³ für Wasserstoff, C₁-C₄-Alkyl oder für einen Rest -NR⁴R⁵ steht, wobei R⁴ und R⁵ unabhängig voneinander Wasserstoff oder einen der für R¹ definieren Alkyl-, Cycloalkyl-, Phenyl- oder Naphthylreste bedeuten und wobei, für den Fall, daß mehr als einer der Reste R¹, R⁴ und/oder R⁵ ungleich Wasserstoff ist, diese Reste die gleiche Bedeutung haben,
in Gegenwart eines inerten Verdünnungsmittels und eines Übergangsmetallkatalysators unter Druck umsetzt.

2. Verfahren nach Anspruch 1, **dadurch gekennzeichnet, daß** man als Verdünnungsmittel eine tertiäre stickstoffbasische Verbindung verwendet.

3. Verfahren nach Anspruch 1 oder 2, **dadurch gekennzeichnet, daß** man als Verdünnungsmittel Chinolin, Isochinolin oder Chinaldin verwendet.

4. Verfahren nach den Ansprüchen 1 bis 3, **dadurch gekennzeichnet, daß** man als Übergangsmetallkatalysator metallisches Kupfer oder anorganische oder organische Kupfersalze verwendet.

5. Verfahren nach den Ansprüchen 1 bis 4, **dadurch gekennzeichnet, daß** man es unter dem sich im geschlossenen System entwickelnden Eigendruck vornimmt.

6. Verfahren nach den Ansprüchen 1 bis 5, **dadurch gekennzeichnet, daß** man es bei 120 bis 250°C vornimmt.

## Claims

1. A process for preparing perylene-3,4-dicarboximides of the formula I 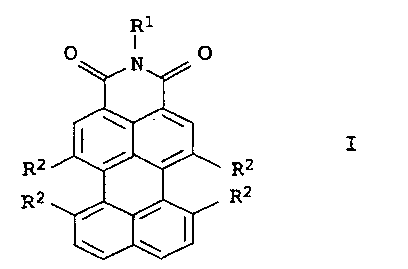 where
R¹ is hydrogen; C₁-C₈-alkyl; C₅-C₈-cycloalkyl; or phenyl or naphthyl, each of which can be substituted by C₁-C₁₀-alkyl, C₁-C₆-alkoxy or cyano; and
R² independently at each occurrence is hydrogen; C₁-C₁₈-alkyl; or aryloxy, arylthio, hetaryloxy or hetarylthio, each of which can be substituted by C₁-C₁₀-alkyl, C₁-C₆-alkoxy or cyano,
which comprises reacting a perylene-3,4,9,10-tetracarboxylic acid or its anhydride of the formula II 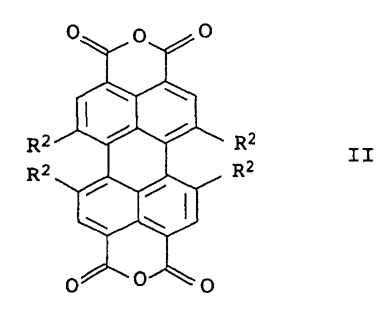 with an amide of the formula III
R3―CO― NHR¹ III
in which R³ is hydrogen, C₁-C₄-alkyl or -NR⁴R⁵ where R⁴ and R⁵ independently are hydrogen or one of the alkyls, cycloalkyls, phenyls or naphthyls defined for R¹ and where if two or more of R¹, R⁴ and R⁵ are other than hydrogen they are identical
in the presence of an inert diluent and a transition metal catalyst and under superatmospheric pressure.

2. A process as claimed in claim 1, wherein the diluent used is a tertiary nitrogen-basic compound.

3. A process as claimed in claim 1 or 2, wherein the diluent used is quinoline, isoquinoline or quinaldine.

4. A process as claimed in any of claims 1 to 3, wherein the transition metal catalyst used is metallic copper or an inorganic or organic copper salt.

5. A process as claimed in any of claims 1 to 4, which is carried out under autogenous pressure.

6. A process as claimed in any of claims 1 to 5, which is carried out at from 120 to 250°C.

## Revendications

1. Procédé de préparation d'imides d'acide pérylène-3,4-dicarboxylique de formule générale I 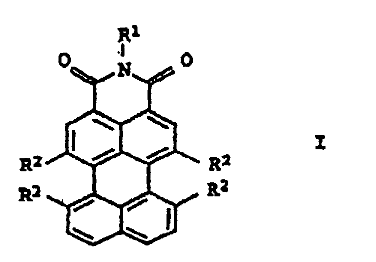 dans laquelle les variables ont la signification suivante:
R¹ représente un atome d'hydrogène ; un groupe alkyle en C₁ à C₈ ; un groupe cycloalkyle en C₅, à C₈, un groupe phényle ou naphtyle qui peut être à chaque fois substitué par un groupe alkyle en C₁ à C₁₀, alcoxy en C₁ à C₆ ou cyano ;
les R² représentent, indépendamment les uns des autres, un atome d'hydrogène ; un groupe alkyle en C₁ à C₁₈ ; un groupe aryloxy, arylthio, hétéroaryloxy ou hétéroarylthio qui peut être à chaque fois substitué par un groupe alkyle en C₁ à C₁₀, alcoxy en C₁ à C₆ ou cyano,
**caractérisé en ce que** l'on fait réagir, sous pression, un acide pérylène-3,4,9,10-tétracarboxylique ou son anhydride de formule II 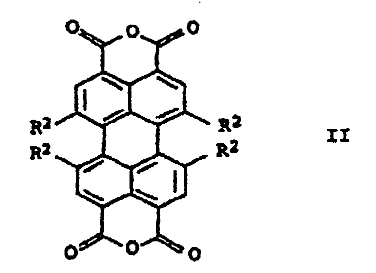 avec un amide de formule III
R³-CO-NHR¹ III
dans laquelle R³ représente un atome d'hydrogène, un groupe alkyle en C₁ à C₄ ou un groupe -NR⁴R⁵, dans lequel R⁴ et R⁵ représentent, indépendamment l'un de l'autre, un atome d'hydrogène ou un des groupes alkyle, cycloalkyle, phényle ou naphtyle définis pour R¹ et dans lequel, dans le cas où plus d'un groupe parmi R¹, R⁴ et/ou R⁵ ne représente pas un atome d'hydrogène, ces groupes ont la même signification,
en présence d'un diluant inerte et d'un catalyseur de métal de transition.

2. Procédé selon la revendication 1, **caractérisé en ce que** l'on utilise, en tant que diluant, un composé d'azote tertiaire basique.

3. Procédé selon la revendication 1 ou 2, **caractérisé en ce que** l'on utilise, en tant que diluant, la quinoléine, l'isoquinoléine ou la quinaldéine.

4. Procédé selon les revendications 1 à 3, **caractérisé en ce que** l'on utilise, en tant que catalyseur de métal de transition, du cuivre métallique ou des sels de cuivre inorganiques ou organiques.

5. Procédé selon les revendications 1 à 4, **caractérisé en ce que** l'on travaille sous la pression autogène se développant dans le système fermé.

6. Procédé selon les revendications 1 à 5, **caractérisé en ce que** l'on travaille à une température allant de 120°c à 250°C.
